# EUROPEAN PATENT APPLICATION

(11) **EP 3 790 023 A1**
(43) Date of publication of application: **10.03.2021**
(21) Application number: 19195018.7
(22) Date of filing: 03.09.2019
(51) Int. Cl.: G16H 80/00, G16H 30/20, A61B 34/10, G06F 3/01

(54) **METHOD FOR ANALYZING MEDICAL IMAGE DATA IN A VIRTUAL MULTI-USER COLLABORATION, A COMPUTER PROGRAM, A USER INTERFACE AND A SYSTEM**

(71) Applicant: Koninklijke Philips N.V., 5656 AG Eindhoven (NL)
(72) Inventor: SCHRECKENBERG, Marcus, 5656 AE Eindhoven (NL); HITSCHRICH, Niklas, 5656 AE Eindhoven (NL); SCHMUMMERS, Georg, 5656 AE Eindhoven (NL)
(74) Representative: de Haan, Poul Erik

(57) **Abstract**

There is provided a method for analyzing medical image data (34) in a virtual multi-user collaboration, wherein the medical image data (34) is analysed by at least two users (A, N,C, S), each user having his/her own workspace (30), wherein the workspace is a VR- and/or AR- and/or MR-, the method including the steps of providing medical image data (34) including 3D or 4D image information, loading the medical image data (34) into the workspace (30) of each user so as to simultaneously display a visualization of the medical image data (34) to each user, allowing each user to individually and independently from each other change the visualization of the medical image data (34), so as to obtain an individual visualization of the medical image data (34) in each workspace (30) pertaining to each user, allowing at least one user to execute an analyzing process of the medical image data (34) in his/her workspace, displaying the result of the analyzing process in the workspace (30) in which the analyzing process was carried out, and synchronizing the result of the analyzing process in real-time with the at least one other workspace (30) such that each workspace (30) displays the result of the analyzing process in the respective individual visualization of the medical image data (34). Further, there is provided a computer program relating to the above method. In addition, a user interface and a system used during execution of the above method are provided.

## Description

### FIELD OF THE INVENTION

The present invention relates to a method for analyzing medical image data in a virtual multi-user collaboration, a computer program related to the method, a user interface used in the method and a system configured to perform the method.

### BACKGROUND OF THE INVENTION

Medical imaging techniques provide three-dimension (3D) - or even four-dimensional (4D) - medical image data of the human or animal body. However, the image data is usually viewed and analyzed on two-dimensional (2D) screens. Therefore, there is an omnipresent risk to misinterpret the relative spatial relationships between anatomical structures represented on medical image data provided in 3D or 4D, when the medical image data is analyzed and interpreted on 2D screens. Specifically, when a team of specialists consisting of different persons are simultaneously analyzing the same medical image data, the risk of misunderstanding and misinterpretation of spatial relationships, resulting in wrong clinical decisions, is high.

Latest developments in the field of microprocessors, cameras and sensors allow for affordable consumer ready VR headsets which are widely accepted by the mass-market. Virtual Reality (VR) technology provides users with a complete immersion in a virtual environment and a stereoscopic 3D representation of 3D content. Driven mostly by the gaming and entertainment industry, the highly-intuitive control via tracked headset and hand controllers (combined 18 degree of freedom) allows a very natural interaction with virtual objects, which in turn leads to a greater ease of use and accessibility. Existing multi-user VR applications are avatar-based in which several users can be in the same scene at the same time to, e.g., manipulate a model of an object. Unfortunately, these avatar-based multiuser solutions are not well matched to more demanding requirements of professional medical VR collaboration, in which a meaningful and time-efficient assessment of virtual 3D datasets, objects (e.g., cut planes) and measurements is required. In addition, the known techniques seem to be inappropriate for professional users (e.g. clinicians) requiring to individually analyze and evaluate the 3D content. Therefore, it would be desirable to have a multiuser VR collaboration solution capable of exploiting the full potential of VR and enabling several clinicians to efficiently and simultaneously work on a same 3D dataset with all the advantages that the VR environment offers. Further, it would be desirable, in order to enhance the collaboration efficiency of a team of professional users, to interactively and automatically share the result of analysis and evaluation made by each user.

David W. Shattuck discloses in "VR-framework for multiuser virtual reality environment for visualizing neuroimaging data" in Healthcare Technology Letters; Received on 13th August 2018; accepted on 20th August 2018, that a VR framework can support multiple simultaneous users operating in the same virtual space using a client-server model, where one user operates as the server and has control over the system display. The VR environment for each user is driven by an individual network computer connected to the user's headset. Each client computer has access to a copy of the data to be displayed, which can be either on a local drive or a shared network drive. The server listens for clients on a TCP/IP port and then establishes a network connection with each client. Once a client is connected, the server will routinely transmit a small data object that contains viewing state information, which each client uses to update its display. This object includes data necessary to synchronize the view, such as rotation, scale, volume position, and cutting plane. Each client transmits pose information for its headsets and controllers to the server, which broadcasts these to the other clients for display. Models of the headsets and controllers for the other users in the system are rendered in each individual client view. This enables users to interact directly with each other and also helps to avoid real-world collisions between users operating in a shared physical space.

Klaus Engel discloses in *"Texture-based Volume Visualization for Multiple Users on the World Wide Web"* a texture-based volume visualization tool, which permits remote access to radiological data and supports multi-user environments. The tool allows the shared viewing and manipulation of three-dimensional medical volume datasets in a heterogeneous network. Volume datasets are transferred from a server to different client machines and locally visualized using a JAVA-enabled web-browser. In order to reduce network traffic, a data reduction and compression scheme is proposed. The application allows view dependent and orthogonal clipping planes, which can be moved interactively. On the client side, the users are able to join a visualization session and to get the same view onto the volume dataset by synchronizing the viewpoint and any other visualization parameter. Interesting parts of the dataset are marked for other users by placing a tag into the visualization. In order to support collaborative work, users communicate with a chat applet, or by using any existing video conferencing tool.

Dieter Schmalstieg discloses in *"Bridging Multiple User Interface Dimensions with Augmented Reality"* an experimental user interface system, which uses collaborative augmented reality to incorporate true 3D interaction into a productivity environment. This concept is extended to bridge multiple user interface dimensions by including multiple users, multiple host platforms, multiple display types, multiple concurrent applications, and a multi-context (i.e., 3D document) interface into a heterogeneous distributed environment. Contexts encapsulate a live application together with 3D (visual) and other data, while locales are used to organize geometric reference systems. By separating geometric relationships (locales) from semantic relationships (contexts), one achieves a great amount of flexibility in the configuration of displays. Multiple users are working on separate hosts. They can share contexts, but can layout the context representations (3D-windows) arbitrarily according to screen format and personal preferences. This is made possible by defining separate locales, as the position of 3D-windows is not shared across locale boundaries. In other words, one shared object can be viewed by multiple users from different perspectives.

EP 3496046 A1 concerns a method for displaying medical image data on at least one display medium for a group of at least two interactive viewers, having the steps of providing medical image data which contain at least one 3D or 4D image data set of a specific examination area of a patient; and providing a possibility for displaying the medical image data in an interactive, virtual environment, wherein each interactive viewer has its own virtual position. Further, each interactive viewer can change his/her virtual position and optionally his/her viewing angle independently of the other viewers.

However, the problems mentioned above remain; in other words, a collaboration efficiency is not significantly enhanced because analyzing results of the users have to be actively shared among the users based on a command of one or more users. As a result, repetitive working steps are necessary to provide users with relevant information obtained by other users.

### OBJECT OF THE INVENTION

It is therefore an object of the invention to provide a method for analyzing medical image data in a virtual multi-user collaboration in which each user has full control over the visualization of the medical image data in his/her workspace while at the same time the collaboration efficiency is kept high, that is, a team of professional users are able to work simultaneously on the same medical image data. According to a further object of the invention, a selectable part of the individual work can be automatically shared among the users. It is also an object of the present invention to provide a respective computer program, a user interface configured to be used in executing the method and a system for virtual collaboration for interaction with 3D medical image data allowing the user to still maintain full control over the visualization of the medical image data.

### SUMMARY OF THE INVENTION

To better address one or more of the above-identified concerns, in a first aspect of the invention a method for analyzing medical image data is presented in claim 1. Useful embodiments are set out in the dependent claims.

In accordance with this first aspect, the invention is directed to a method for analyzing medical image data in a virtual multi-user collaboration, wherein
the medical image data is analysed by at least two users,
each user having his/her own workspace, wherein the workspace is a XR-workspace.
the method including the steps of:
providing medical image data including 3D or 4D image information,
loading the medical image data into the workspace of each user so as to simultaneously display a visualization of the medical image data to each user,
allowing each user to individually and independently from each other change the visualization of the medical image data, so as to obtain an individual visualization of the medical image data in each workspace pertaining to each user,
allowing at least one user to execute an analyzing process of the medical image data in his/her workspace,
displaying the result of the analyzing process in the workspace in which the analyzing process was carried out, and
synchronizing the result of the analyzing process in real-time with the at least one other workspace such that each workspace displays the result of the analyzing process in the respective individual visualization of the medical image data.

In the context of this invention, the term XR stands for X reality or Cross Reality, which is a generic term comprising at least Virtual Reality (VR), Augmented Reality (AR), Mixed Reality (MR), and Cinematic Reality (CR). XR maybe defined as a technology-implemented experience of a virtual environment, often combined with real world objects, or a combination of a real world environment combined with virtual environments or objects. X Reality encompasses a wide spectrum of hardware and software, including sensory interfaces, applications, and infrastructures, that enable content creation for virtual reality (VR), mixed reality (MR), augmented reality (AR), cinematic reality (CR). With these tools, users generate new forms of reality by bringing digital objects into the physical world and bringing physical world objects into the digital world. XR is used here to refer to various technologies including VR, AR or MR. In many cases, in order to provide an X Reality, a computer-generated visualisation providing a truly three-dimensional experience of the depicted structures is implemented, in particular by using screens or glasses showing a slightly different image to each eye. Further, the user may often interact with the XR by gestures and other movements, e.g. walk around in the XR and grab virtual objects. The XR of the invention may provide in particular visual feedback, but may also allow other types of sensory feedback, such as auditory or haptic feedback to a user.

In VR, the real environment is usually not visible to the user, it is completely overlaid with a virtual world. This effect is commonly created by VR headsets consisting of a head-mounted display with a small screen in front of the eyes, but can also be created through specially designed rooms with multiple large screens. A person using virtual reality equipment is able to look around the artificial world, move around in it, and interact with virtual features or items.

Generally, Mixed reality (MR) is the merging of real and virtual worlds to produce new environments and visualizations where physical and digital objects co-exist and interact in real time. More specifically, MR may be defined as an experience of the real environment combined with virtual objects which however is created by technology, for example a VR headset using its cameras to create a virtual reality which corresponds at least in parts to the real environment. Thus, virtual objects can be overlaid on the real environment at their correct position, they may even hide real objects and vice versa. In AR, the real world is still visible, and virtual objects are superposed (i.e. overlaid) over the real environment. This effect may be created by special glasses such as Microsoft HoloLens which allows the user to see the real environment, but which also uses cameras to form a 3D model of such real environment so that virtual objects can be overlaid over the real environment via the glasses.

Thus MR or AR may include the real environment as a background in front of which the visualization is displayed. For example, two users present in the same room and each wearing AR glasses may interact with each other in the real world and in the AR, while both users have their own individual view of the medical image data (i.e. each user has his/her own visualisation of the medical image data).

The visualisation may be an effigy (i.e. an image) of a real object (e.g. a human or animal heart). That is, the visualisation may be a model representing the real object, wherein parameters of the visualisation may be changed with respect to the real object (e.g. the size, contrast, colour, partly enlarged regions etc.). In order to visualize the medical image data, the technique of volume ray casting may be used. In this technique, a ray is generated for each desired image pixel. Using a simple camera model, the ray starts at the centre of protection of the camera (usually the viewing position or eye point) and passes through the image pixel on an imaginary image plane floating between the camera and the volume to be rendered. Then the ray is sampled at regular or adapted intervals throughout the volume. The data is interpolated at each sample point, a transfer function applied to form an RGBA sample, the result added onto the accumulated RGBA of the ray, and the process repeated until the ray exits the volume. The process is repeated for every pixel on the screen to form the completed visualization.

For example, the medical image data maybe visualized using volume rendering. The volume rendering of the visualisation may be performed by techniques described in "The Visualization Handbook", edited by Charles H. Hansen and Christopher R. Johnson, Elsevier Butterworth Heinemann 2005, especially in the Chapter "Overview of Volume Rendering" by Arie Kaufmann starting on p. 127, and which is incorporated herein by reference.

Technically, the XR may be realised by presenting stereo images to the user, i.e. each eye sees a different image, so that the brain will put together the two different images to a true three-dimensional scene. Such binocular images may be presented on any XR display, such as a VR headset, AR glasses or a multi-projected environment, or a screen showing the two images intermittently, in connection with shutter glasses. In the XR, the visualization may be displayed by stereoscopic rendering: Therein, the visualisation is calculated twice, for two viewing positions having a slight spatial offset, i.e. one viewing position for the left eye and one for the right eye. When the two thus calculated visualisations are shown to the user one on each eye, e.g. on a VR headset, the user gains a truly three-dimensional (VR) impression. Thereby, the visualization can be converted, viewed and analysed in XR. The XR enables the user using the XR to "look around" the artificial world, move around in it, and interact with virtual objects, features or items. The effect is commonly created by XR headsets comprising a head-mounted display with a small screen in front of each eye, but can also be created through specially designed rooms with multiple large screens. In order for the user to move around in the XR, position and orientation information have to be transmitted by the headset to the electronic device (e. g. computer) generating the XR, so that the visualisation is moving in coherence with head movements of the user.

The medical image data may be dynamically rendered (e.g. volume rendered or surface rendered) so as to be visualized in each XR-workspace as the visualization. In more detail, volume rendering may be based on spatial intensity data (i.e. voxel data). Depending on the resolution of the medical image data there may be an intensity data for each point in space or for a region in space. In other words, in volume rendering each available image data information is rendered, which results in a considerable high computing capacity that is needed. On the other hand, in surface rendering, only one "layer" (i.e. the visible surface) is rendered, wherein the image data existing behind the layer is not rendered. The surface rendered medical image data may be a computer graphical model consisting of a plurality of triangular shaped surfaces. As a result, for surface rendering less computing capacity is needed. In order to attain an increased efficiency during the rendering process while still providing a sufficient visualized data density, both techniques may be combined such that a region of interest may be rendered using the method of volume rendering and peripheral regions may be rendered using the method of surface rendering.

In a preferred embodiment, the medical image data maybe visualized using a volume rendered object which has the advantage that it is suitable for more complex anatomies or highly individual structures like valve leaflet cusps, stenosis, calcifications, bio-protheses, ruptured chordae etc. Alternatively or additionally, the medical image data may be visualized using a dynamic, computer-generated model of at least a part of an anatomical structure. Such models have the advantages that they show a simpler version/abstraction of the anatomy, make it easier to navigate and interpret the anatomy, and are not very much dependent on the medical image data quality. The model (i.e. the simplification of the anatomical structure) may be a triangulated surface model of a particular interface within the anatomical structure, for example the blood-tissue interface of a blood vessel or a heart chamber. The model may comprise a number of points spanning a line or a surface for each frame. It may also be a mathematical model, for example a parametrised model, such as a surface or volume spanned by spline curves. The model is dynamic, i.e. it follows the movement of the anatomical structure across the time period. The purpose of the dynamic model is to visualise at least a part of the anatomical structure, for example one or several chambers of the moving heart, without obstructing the view of the user with too much detail. Therefore, such simplified models are useful in providing an orientation to the user, for example when planning an intervention or making measurements on a particular part of the anatomy. The 3D visualization of the dynamic 3D model is typically a rendering of a dynamic shape or surface model, wherein the rendering may be done by techniques available from computer graphics, including shading, ray casting, ambient occlusion etc.. The volume rendering may be performed by any volume rendering technique known in the art for example as described in US 2005/0253841 A1, incorporated herein by reference.

Further, the medical image data may include information of an anatomical structure which may be an organ or part of an organ of the human or animal body such as heart, but may be also a blood vessel or a bone structure. In more detail, the medical image data may include a 3D scatter plot including points within a 3D coordinate system, wherein each point has its own x, y and z component within the 3D coordinate system. In addition to the above-outlined, the medical image data may include digital image data e.g. in DICOM standard i.e. containing a three-dimensional array of voxels, each voxel containing a grey scale value. Such medical data (3D medical images) may be obtained from a field of view containing the dynamic anatomical structure using a medical imaging modality such as MR, computed tomography (CT), position emission tomography (PET), or ultrasound (US). In case the anatomical structure is the heart, ultrasound and in particular three-dimensional echocardiography may be advantageously used. That is, the different medical image data may be derived from ultrasound images having different frequencies, computed tomography having different acceleration voltages or images including contrast medium or not. In addition, the medical image data may include 4D medical image data, wherein the fourth dimension is time. One 3D image from the time sequence of 3D images forming a 4D medical image may also be called a "frame" in the following. That is, 4D medical image data includes the visualization of 3D images across time, which means that a sequence of visualizations is shown dynamically, at a frame rate of e.g. 60-100 visualizations per second. That is, 4D medical image data may be visualized as animated movie (i.e. in a cine-mode) so as to visualize the operation of a thorax of a patient, for example. If, 4D medical image data are visualized, the sequence may be rendered in a movie-like manner. The 3D images may be acquired with a frame rate of e.g. 5 - 100, preferably more than30 images per second, so as to allow a smooth representation of the dynamically moving anatomical structure. Medical image data may include further medical and/or administrative information e.g. information of the patient, the currently executed therapy etc.. Medical image data may include several image data which are preferably registered to each other in space, so they can be overlaid with each other, each user may select which one should be displayed in his/her workspace.

The medical imaging modality may be capable of attaining up to 30 volumes per second (i.e. medical image data representing a region under inspection), therefore, the rendered 3D object maybe updated each time new medical image data are available. In other words, the rendered 3D object maybe dynamically updated. This is particularly useful, if a patient is simultaneously examined while the collaboration takes place, in cases in which the patient is in an emergency, for example.

It is to be noted that each user shares the same medical image data with all other users. In addition, the result of the analyzing process is also shared by all other users because the result is coupled to the medical image data shared by all users. On the other hand, the visualization displayed in each workspace may be individually changed by a user so as to attain an individual view of the medical image data. In other words, according to the present invention, the medical image data and the result of the analyzing process are identical and shared by all users, wherein the display of this content (i.e. the visualization of the medical image data and the result) is subjected to the user himself/herself. As a result, according to the present invention each user has the maximum freedom to inspect the medical image data as he/she wants, while at the same time the result of the analyzing process of other users is displayed in his/her personalized visualization (i.e. in his/her individual view of the medical image data) in real-time. Hence, the medical image data and the result of the analyzing process are decoupled from the visualisation of the medical image data in each workspace. In other words, each user has his/her individual view of the medical image data, while at the same time the medical image data and the result are the same for all users.

The user maybe a participant in the collaboration session, specifically a doctor, for example, an interdisciplinary heart team (IHT) may use the present invention, wherein the teams may consist of an interventional cardiologist, cardiovascular surgeon, care/nurse coordinator, OR/Cath lab nurse, imaging specialist, cardiac anaesthesiologist. Each user uses his/her own XR workspace. Alternately, at least two users may share the same workspace, for instance in a scenario in which one user is the teacher and another user is a student.

Each of the XR-workspace may be physically connected to another XR-workspace via cable or wireless. Each workspace may have an own personal computer including a processor. Each computer may work as a client-computer. Alternatively, at least some of the workspaces may be part of one client-computer. That is, XR workspaces may be located at different locations or may be in the same room, for example.

The step of allowing each user to individually and independently from each other change the visualization in a user" s own XR workspace is executed in such a way that none of the other users notices how the visualization (i.e. the individual view of the medical image data) is changed in another XR workspace. Specifically, a viewing position and a viewing direction in space may be changed. Further, the position or orientation of a plane cutting through the 3D medical image data (herein referred to as "cutplane") may be individually adjusted, as well as the mode of displaying such plane in the visualization. For example, the user may select one of several so-called "cutplane" modes: In one mode, no cutplane is displayed, in another, the cutplane cuts through a 3D image volume and is overlaid over the image content. In another mode, the cutplane displays a corresponding multi planar reconstruction (MPR) of the 3D image data. Further, the opacity and colour of the visualisation may be changed. Generally, visualization parameters of the visualization may be changed, e.g. volume rendering parameters. Specifically, the visualization parameters may include thresholds, opacity, contrast etc. Further, the visualization may be adjusted "live", that is, with an immediate effect while watching the visualization.

According to an aspect of the present invention the result of the analyzing process is synchronized in real-time with the at least one other workspace such that each workspace displays the result of the analyzing process in the respective individual visualization of the medical image data. Since the result of the analyzing process belongs to the medical image data which are shared by all users (refer also to the above outlined), the synchronization in this case means the transmission of data attained in the analyzing process from the respective workspace to the shared medical image data. That is, the results of the analyzing process are visible to all other users at the same time as they are made. In other words, immediately after the analyzing process is executed, each of the other users may see the results in their own individual visualization of the medical image data. For example, each user sees the annotations of the other users live in his own visualisation of the medical image data. This makes "handing over" the medical image data between multiple users obsolete and therefore results in fewer interactions between users and a faster sharing of measurement results and annotations.

According to an embodiment, there is no indication displayed within the workspace in addition to the visualization that indicates a position of other users and/or a viewing direction thereof e.g. by displaying an "avatar" of each user. Therefore, there is no risk that a part of the visualization is covered and/or hindered by such indication.

For example, the execution of the analyzing process may include the selection of a plane in the visualization. The process then preferably comprises a step of displaying a multi planar reconstruction (MPR) of the selected plane of the visualization, in particular at the position in the three-dimensional visualisation environment corresponding to the selected plane. A multi planar reconstruction is an image reconstructed from several original image planes. In CT for example, a stack of usually transversal images is acquired. Further, in the analyzing process, the user may measure the diameter of the mitral valve and accordingly select the best fitting valve from a library. Thus, if a sectional plane intersecting the stack of images at a different orientation than transverse is to be viewed, the user may select the desired orientation, and an MPR is created by e.g. interpolating from the respective nearest pixels in the various transverse slices. Displaying an MPR in addition to the visualization allows the user to view the anatomical structure in more detail. In the virtual reality environment, thanks to the 18 degrees of freedom (XR headset and two controllers), the correct positioning of a grippable MPR plane in the 3D volume is very fast and verifiable, and measurements on the MPR plane or within the visualization become more precise and reliable.

For example, the result of the analyzing process may be at least one measurement result of features of the visualization of the medical image data such as distances, diameters, thicknesses etc. In more detail, the measurement result may be displayed by a first dot at a first location from which a distance is to be measured (starting point) and a second dot at a second location to which the distance is to be measured (end point) and a line connecting the first dot and the second dot. Specifically, the result of the analyzing process may be three-dimensional notes such as a 3D freehand line that maybe drawn by a user within his/her workspace. In other words, the result of the analyzing process may be no planar objects but 3D objects that extends in any one of the three dimensions within the 3D coordinate system. For example, the user may retrace the flow of blood through a heart or follow the course of the coronary arteries.

The first aspect of the present invention provides the advantage that each user can generate his/her own preferred visualization of the medical image data, in other words, each user has his/her own individual view of the medical image data. Further, each user may analyse and view the visualization of the medical image data in his own way (e.g. individual viewing directions, cutplane positions, thresholds and speed), that is, some users may need more time for specific procedures as compared to other users. Since there is no avatar in the visualization, there is no occurrence of hiding parts of the medical image data behind such avatars. In addition, unexperienced user may follow results made by another user in real-time in order to better understand a subject matter they will not understand by themselves.

Moreover, the XR provides the advantage that the user may view and analyse the visualization with great confidence, since he obtains a truly three-dimensional view of the anatomical structure represented by the medical image data. Further, since he can walk around it and possibly even into it, he can have the visualised object (e.g. the visualisation of the human heart) displayed in huge magnification, so as to completely fill the space in front of the user. Therefore, the user has a particularly good overview and may take measurements with great accuracy. Further, the handling of user input events is particularly easy and intuitive in XR. Actions such as rotating the objects and/or adjusting the settings of the visualization, which may be quite tricky on a two-dimensional screen, are very intuitive and fast in a XR preferably using XR controllers.

In an embodiment, the medical image data include images, preferably 4D images, of a human heart.

The invention may find particular use in planning minimally invasive heart surgery, such as surgery on a heart valve or a heart valve replacement. New minimally invasive methods like transcatheter valve replacement can be used for patients who were formerly considered inoperable and/or not suited for open-heart surgery. Some transcatheter valve replacements (e.g. TAVR) use a fully collapsible bioprosthetic valve. However, it is crucial for the success of these interventions that the existing pathology/geometry is analysed and understood completely and that the new valve is carefully selected, sized and positioned to ensure that it is working properly and not obstructing the left ventricular outflow tract (LVOT) or coronary arteries. This is particularly true for valve-in-valve (ViV) interventions. Thereby, a dysfunctional valve - sometimes a bioprosthetic mitral valve - is replaced by a new valve in a minimally invasive ViV procedure. Thereby, the new valve is positioned inside the old valve, replacing the old valve while it unfolds. Therefore, it is crucial that the valve should be positioned correctly and have the correct size. In particular, it is important that the new mitral valve does not obstruct the LVOT. Therefore, for valve in valve intervention planning, the medical image data contains the mitral valve, the LVOT and the left ventricle.

In a preferred embodiment of the present invention, the displaying of the result of the analyzing process may be selectively and individually enabled and disabled by a user in his/her workspace.

Each result of the analyzing process may be positioned at a specific position within the 3D coordinate system (i.e. the position may be defined by x, y and z coordinates) in which the medical image data is defined. That is, the result may be located at a specific position in relation to the medical image data. Because the medical image data and the result are shared by all users, the result is displayed within each workspace regardless in which way the user has individually changed the visualization (i.e. his/her individual view of the medical image data). The result may be visualized by relatively thin lines such that it is less likely that the result covers other objects displayed in the workspace. Nevertheless, there may be a situation in which the result covers a part of the visualization and/or other results. Therefore, according to the embodiment of the present invention, the result may be disabled so as to vanish and provide a free view onto objects positioned behind the result.

Since each of the user may have a different focus with respect to a region of interest, it is important that each user may individually decide which result hinders a sufficient view onto the region of interest and thus shall be disabled. That is, each user may selectively disable results made by himself/herself or other users. Further, enabling or disabling the result may have no influence on the other at least one workspace such that the enabling and disabling of the result may be executed independently in each workspace. In addition, the method may allow a user to adjust the transparency of the result so as to see objects placed behind the result through the result. Particularly, once the result is disabled, a user may enable the result again so as to again see the result within his/her workspace. For this reason, the result, in a disabled state, maybe indicated by a small arrow or similar to provide the user with the information where the result was originally placed. As a result, the user may easily regain the result for enabling the same again.

According to this embodiment, the visualization in each workspace may be further individualized in that each user may individually adjust both the visualization (i.e. the individual view of the medical image data) and the result exactly in a way that satisfies his/her requirements with respect to workability.

In a further preferred embodiment of the present invention, the at least one workspace may be an AR-workspace. That is, both the visualization and the result are displayed in the workspace while the real environment is still visible as a background of the workspace. For example, the real environment may be the surrounding area of the user (e.g. an operating room or a doctor's office). Further according to an embodiment, at least one visualisation parameter within the AR-workspace, in particular a transparency and/or a colour of the visualization of the medical image data and/or the result of the analyzing process may be adjusted automatically, so as to allow the user to view the visualization of the medical image data and/or the result of the analyzing process superposed on the real environment with a target contrast.

In more detail, visualisation parameters may be values defining the way in which the medical image data and/or the result is/are visualized. Therefore, by changing these values the visualization also changes accordingly. For example, the visualisation parameters may include the transparency value, that is, the value of lucency of an object. For example, if an object has a low transparency the region covered by the object is not visible. On the other hand, if the object has high transparency the region covered by the object might be at least slightly visible. Further, visualisation parameters may include the colour of the visualization, that is, the hue or tone of the visualization. In more detail, hue is one of the main properties (also called colour appearance parameter) of a colour. Hue can typically be represented quantitatively by a single number, often corresponding to an angular position around a central or neutral point or axis on a colour space coordinate diagram (such as a chromaticity diagram) or colour wheel, or by its dominant wavelength or that of its complementary colour. The other colour appearance parameters are colourfulness, saturation (also known as intensity or chroma), lightness, and brightness. In the present embodiment inter alia, these parameters may be automatically adjusted in order to attain the target contrast within the workspace. The target contrast may be also referred to as an optimal contrast. The contrast within the workspace may be defined as the difference between the brightest point and the darkest point within the workspace, wherein within the AR-workspace the real environment is considered, too. In addition, the contrast within the workspace may be also defined as the contrast ratio or dynamic range. The visualization of the medical image data and/or the result may be adjusted in such a way that the contrast within the AR-workspace amounts to the target contrast. In other words, if the real environment (background) is relatively bright, the brightness of the visualization and/or of the result is/are also increased so as to reduce the whole contrast to the target contrast. The target contrast may be a predefined value, which is defined as being most appropriate with respect to detectability and conspicuousness of the visualisation. Alternatively, the target contrast maybe individually adjusted and/or set by the user so as to satisfy his/her personal predilection.

According to an embodiment, each workspace may have its own virtual environment in which the visualization of the medical image data and the result of the analyzing process are displayed. The virtual environment may be the background in which the visualization is displayed. Further, according the embodiment the method may further include the step of allowing each user to individually and independently adjust a visualization parameter of the virtual environment so as to adjust a contrast within the workspace, preferably by setting a transparency and/or a colour of the virtual environment. The virtual environment may be the background in front of which the visualization is positioned. In other words, the virtual environment may be the surrounding which surrounds the visualization. For example, in case the VR-workspace is used, the virtual environment may include a coloured surface that has a defined colour and brightness so as to provide a pleasant working environment for the user. That is, the virtual environment may be adjusted so as to provide a contrast within the workspace that amounts to the target contrast. In addition, the user may individually adjust the virtual environment. Further, in case the AR-workspace is used the real environment is also visible as a background within the workspace. However, even the AR-workspace may have a virtual environment that has a specific transparency so as to provide the possibility for the user to see the real environment, too. However, the value of transparency of the virtual environment may be adjustable so as to shadow a very bright real environment such that the contrast within the workspace amounts to the target contrast. This is particularly useful when the AR-workspace is used in a relatively bright environment such as an operating room. In other words, the virtual environment of an AR-workspace allows the user to automatically adapt the AR-workspace to conditions of the real environment while still offering the possibility to recognize the real environment. Moreover, the AR-workspace may be easily adapted to varying light conditions of the real environment. As a result, the AR-workspace is appropriately usable regardless of the light conditions of the real environment.

Advantageously, the virtual environment may include at least one virtual control element. For example, the virtual control element may be a slide bar and/or a button. The control element may be used to execute steps of the method e.g. changing the visualisation and/or to implement further administrative processes like saving an image, loading medical image data, communicate with other users etc. Further, the control element may provide a drop-down menu in which several predefined control commands may be listed.

In a further preferred embodiment, the step of allowing each user to individually and independently change the visualization of the medical image data may include the use of a VR controller in order for the user to interact with virtual features in the workspace. Specifically, in order to execute the change of the visualization using hand gestures, preferably by grabbing an object in the workspace. In the XR workspace, a user wearing a XR headset and holding at least one controller in one hand (preferably a controller in each hand) sees in the controller together with the visualisation. The controller may be a hand held device including further operating elements e.g. buttons, track ball, touchpad, etc.. In case a VR-workspace is used, an indication (e.g. a virtual controller) of the controller within the VR-workspace may be depicted so as to inform the user where the controller is positioned within the VR-workspace. Preferably, he can also see the virtual controllers at the positions and orientations corresponding to the current hand positions and orientations. Thus, the XR workspace provides the possibility for the user to move the controllers towards the visualisation, grab it by pressing a particular button, and move and/or rotate the visualised object with the movement of his hands, like he would with a real-world object. Thereby, the users have 18 degrees of freedom (six degrees of freedom, namely three rotational and three translational degrees of freedom for each of the XR headset and the two controllers) to correctly and intuitively view and analyse the visualised object. This closely resembles the natural way of interacting with objects. A movement of the controller may be tracked and a correspondent movement of the indication is visualized within the VR-workspace. On the other hand, in case the AR-workspace is used, the real controller may be still visible. However, even in the AR-workspace a virtual controller may be visualized in order to improve the conspicuity of the controller.

The position information may be attained by at least one sensor e.g. an acceleration sensor located within the controller. More commonly, the position(s) and/or orientation(s) of the controller(s) is/are tracked by the cameras on the VR headset. The data outputted by the sensor or camera may be inputted to a processor that processes the data so as to control the commands executed by operations of the controller and the visualization of the virtual controller. Further, dependent on the output of the sensor the processor may determine whether a gesture is performed slow or fast. Further, a rotation movement may be detected. As a result, the processor may perform processes accordingly.

Operations executed by the controller may include zoom in/zoom out the visualisation, scale down/ scale up the visualisations within the workspace, adjust visualisation parameters and rendering settings/parameters, and/or grab the displayed objects, in particular the visualisation of the medical image data.

Additionally, the controller may receive hand gestures made by the user, for example, a grabbing gesture, a zoom-in gesture (approaching of two fingers), zoom-out gesture (departing of two fingers) a wipe gesture etc.. Further, the visualization may be virtually grabbed using the controller and rotated or moved, for example. In a XR workspace the real controller may be visualized as a virtual controller allowing a user at least to grab and move an object within the workspace by hand gestures. "Move" in this context may also mean rotate. The controller may also allow a user the change the size and/or shape of objects within the workspace, specifically, of the visualization. According to a useful embodiment, the controller as described above allows a user to change any one or several of the position, size, shape and/or orientation of the visualization. In particular, the position and/or orientation of the visualization may be adjusted (i.e. the individual view of the medical image data). However, in a useful embodiment also the size of the visualization may be changed to better analyse the visualization. Preferably, such adjustments are made by using the controller by grabbing the visualization, and changing its position, size and/or orientation by hand gestures, as one would a real-world object. According to a useful embodiment, a controller allows a user to adjust parameters by means of gesture control. For example, the user selects a certain parameter by touching it using hand movement in the XR workspace. He/she may then use gestures to e.g. actuate a virtual slider, or simply move the controller horizontally (or vertically) to adjust the parameter without reference to any slider. Suitable parameters are related to the visualisation and may be selected from a volume rendering threshold, smoothing, lighting intensity, size, opacity of a visualised object, starting and holding the cine-mode etc.

As a result, the user may intuitively operate within the AR/VR-workspace like moving objects or operate operation elements such that the efficiency of such collaborations is increased.

Further, in useful embodiments the workspace may comprise a lamp which the user may grab and move within the workspace, so as to influence the lighting of the visualization. In useful embodiments, also the brightness of the scene, in particular the brightness of a movable lamp, may be adjusted. Further, by adjusting the position of the lamp, the direction in which the visualization is illuminated may be changed. This is particularly useful in order to illuminate cavities of the visualized anatomy.

Preferably, the step of allowing each user to individually and independently change the visualization of the medical image data includes manipulating the visualization so as to rotate the visualization, cut away a part of the visualization, change rendering parameters of the visualization, change image settings of the visualization, change a contrast of the visualization, change voxel intensity thresholds of the visualization and/or change a size of the visualization. In other words, the visualisation is manipulated e.g. is rotated if the user wants to see another part or region of the visualization. That is, each user has his/her own individual view of the medical image data. In the same way each user may individually cut away a part of the visualisation in order to attain a visualisation in which only the parts of the visualization are displayed that are of interest for the specific user. The rendering parameters of the visualization may include the kind of rendering (i.e. volume rendering and/or surface rendering) and the image settings may include a colour of surfaces and shading options (i.e. the position of a light source in the space). Further, a user may set settings of his individual visualization such that the medical image data is visualized in slices or in other forms. Further, there may be presets of different visualizations in which the medical image data are displayed such as, a plan view, a right front view, a left front view a rear view, button view and a perspective view. The presets may be set in advance or may be predefined by each user.

Advantageously, at least one user may adopt the change(s) of the visualization of the medical image data made by another user. That is, by adopting the visualization of another user (i.e. the individual view of the medical image data), the visualization is transferred to at least one other user's workspace such that at least two users have the same individual view of the medical image data. This procedure may be particularly useful, if there is an experienced user (e.g. teacher) who currently explains something to other users (e.g. to students). As a result, the other user may be in a position to learn from the more experienced user. In other words, the other user(s) is/are provided with the same individual view of the medical image data as the experienced user. The switch from one individual view to another one may be continuously executed so as to show the user in which way the individual view is changed. Alternatively, the switch between the individual views may be illustrated by visualizing the switch in a bird's-eye view, that is, the change between the both individual views is illustrated in a top view.

Alternatively, one user may force at least one other user to adopt his/her change(s) of the visualization of the medical image data. That is, the user may force other users to see exactly that what he/she sees (i.e. having the same individual view), this may be specifically appropriate in courses in which one user wants to show other users a specific part of the workflow. Therefore, the efficiency of the collaboration is improved. In a further preferred embodiment only one user is allowed to decide which change(s) of the visualization of the medical image data may be shared with other users (i.e. the user may be the presenter and/or teacher), whereas the other users have restricted functionalities and may not be allowed to share any change(s) of the visualization of the medical image data (e.g. the users may be participants and/or students).

In a further preferred embodiment, the step of allowing at least one user to execute an analyzing process of the medical image data in his/her workspace may further include taking 3D measurements, executing MPR-mode and/or inserting annotation. That is, the result of the analyzing process may be one or more annotation(s) related to specific aspects of the visualisation such as background information of previously executed therapies, comments of users related to an abnormality etc.. Each result may be visualized by thin lines within each of the workspaces in real time because the result belongs to the medical image data that is shared by all users. That is, immediately after the results have been obtained, each user sees the result in his individual view. The 3D measurements may be a measurement in the 3D dimensional space, that is, a distance between two points which differ from each other in each of the x-coordinate, y-coordinate and the z-coordinate. Further, a 3D measurement may involve tracing a non-planar, irregularly shaped object, for example by measuring the distance spanned by several points in space, for example points along an irregularly shaped object such as the mitral annulus. Such non-planar measurements cannot be performed on a 2D screen, but can easily be done in the XR workspace of the invention. MPR-mode may be a multi-planar reconstruction which is a method of two-dimensional image reconstruction. In MPR, frontal, sagittal, oblique or curved sections are calculated from transversal sections and displayed to help the user with anatomical orientation. Oblique sections, for example, are helpful in heart imaging (four-chamber view, short axis sections), curved reconstructions along structures that are themselves curved several times for the representation of vessels (such as the coronary arteries) or ureters. In order to obtain high-quality MPR reconstructions, the medical image data (e.g. obtained by CT) should be acquired overlapping with a small layer thickness. In addition, a small layer thickness should be selected to avoid step artifacts during image reconstruction. The image noise may be reduced by summing up several layers. Thresholding may be an adaption of the threshold boundaries of each voxel in order to better visualize the region of interest.

Preferably, the step of allowing at least one user to execute the analyzing process of the medical image data in his/her workspace may further include positioning at least one model of a medical device, specifically an implant, within the visualization of the medical image data so as to determine its operational position. The model of a medical device may be a computer graphical object. That is, the model of a medical device may be additionally displayed within the XR-workspace. The medical device maybe an implant or any other device that is to be placed within the human or animal body. The computer graphical object is for example a representation of geometric data, e.g. a 3D structure defined by vertices, such as a polyhedron. The computer graphical object is preferably locked to the movement of the anatomical structure, i.e. it is once placed in a particular position and orientation with regard to the visualisation in any one frame. When the user starts the cine-mode, the processor controlling the visualisation remembers the relative position and orientation of the medical device with regard to the visualisation and will keep this relative position and orientation. In the case that the medical device represents a new valve, such new valve can be locked to the movement of the valve annulus, e.g. the mitral annulus. Preferably, this may be done using 3D speckle. Thereby, important dynamic information over the entire heart cycle is delivered, and the valve may be optimally positioned in its operational position (i.e. in its optimal position), thereby avoiding or limiting any obstruction of an outflow. Further, the user may use the controllers to move and tilt the computer graphical object in relation to the visualisation. Thereby, the user can not only measure, but also "try out" a selected implant or implant size, for example a replacement valve, to see if it fits the anatomical feature, e.g. the mitral valve. For example, the user may select the best fitting valve from a library and place the valve - or rather the computer graphical object corresponding the valve - inside the visualisation for an initial inspection. In a useful embodiment, the computer graphical object corresponds to a CAD model of the medical device, e.g. the CAD model used in the design and/or production of the medical device, or more preferably to a simplified model thereof, for example a simplified model a heart valve.

In another embodiment, the computer graphical object looks similar to what the medical device will look like on interventional X-ray images (fluoroscopy images), because minimally invasive interventions are almost always done under fluoroscopy control. Thus, the user may visualise a scene in three dimensions and yet gain an idea on what the implant will look like on the fluoroscopy image. The computer graphical object is preferably three-dimensional, it may be e.g. be a simplified model of an implant, for example in the form of a wire mesh or an object defined by a set of simple surfaces.

According to a preferred embodiment of the present invention the operational position of the model of the medical device is determined by visualizing the medical device dynamically in operation, preferably in combination with the 4D image information. In other words, the 4D medical image data (i.e. a sequence of 3D medical image date e.g. in the cine-mode) are used to visualize the object that is to be investigated e.g. the heart in operation while the model of the medical device (e.g. the artificial mitral valve) is positioned at its intended position. The dynamic movement of the medical device, e.g. during the heartbeat, may be based on tracked 4D image data. For example, specific landmarks interacting with the medical device are tracked over the sequence of 3D image data, and the virtual position of the medical device is adjusted accordingly. Subsequently, the user may investigate how the specific model of the medical device works in combination with the specific anatomical structures under inspection.

In summary, the present invention according to the first aspect provides a location-independent XR collaboration tool that provides an interconnected communication concept in which the medical image data and results of an analyzing process are shared by all users whereas each user still has his/her own individual view of the medical image data (i.e. his/her own duplicate/visualization of the medical image data) within his/her own workspace.

According to a second aspect of the present invention, there is provided a computer program including the features of claim 12. The computer program comprising program code instructions, which, when executed by a processor, enables the processor to carry out the above method. The computer program may be in any code, in particular a code suited for computer graphic applications, in particular for XR programming.

Further, a computer-readable medium comprising the above-defined computer program may be provided. The computer-readable medium may be any digital storage device, such as a USB-stick, hard disk, CD-ROM, SD card or SSD card. Naturally, the computer program need not be stored on such a computer-readable medium to be supplied to costumer, but may be downloadable via the internet.

Preferably, the method according to the invention is executed by a processor which may be incorporated in any electronic device able to control a display, in particular a XR display such as a XR headset or projection display. Such digital device may be a computer, PC, server, television set, tablet computer, smartphone, laptop, hand-held device or the like. The processor may also be part of a cloud computer, workstation, or the control console of a medical image device, in particular an ultrasound scanner.

According to a third aspect of the present invention, there is provided a user interface configured to be used in executing an above-defined method, wherein the user interface includes:
a XR display device, in particular a VR headset, for displaying a visualization of medical image data and a result of an analyzing process in real-time within a workspace to a user,
   wherein the workspace is a XR-workspace, and
   wherein the workspace includes a virtual environment so as to display the visualization of medical image data and the result of the analyzing within the virtual environment, and
a tracked controller configured to be used during execution of the method in order to input commands by gestures of the user, wherein the commands include:
   selectively and individually enable and disable the display of the result of the analyzing process.

Any features of useful embodiments described in connection with the inventive method also apply to the user interface.

A user interface is for example a system comprising at least a screen or display and usually input elements, e.g. a XR controller, and/or buttons or sliders, allowing a user to interact with the content of the display e.g. by adjusting visualisation parameters/settings, zooming, annotating and/or moving or tilting the visualisation.

Preferably, the commands may further include individually and independently adjusting a contrast within the workspace of the user, preferably by setting a transparency and/or a colour of the virtual environment.

According to a fourth aspect of the present invention, there is provided a system for analyzing medical image data in a virtual multi-user collaboration including: a processor configured to carry out the above-defined method, and at least two above-defined user interfaces that are connected to the processor.

Any features of useful embodiments described in connection with the inventive method also apply to the system.

The virtual reality environment may be realized using commercially available VR equipment, such as the HTC VIVE Pro Virtual Reality System, which includes a VR headset, two VR controllers, two position trackers and (made by HTC Corporation, Taoyuan City 330, Taiwan) or the Oculus Rift S (Oculus, Facebook Technologies, LLC). This headset does not require separate position trackers in the room, since the position tracking function is provided by the headset itself.

### SHORT DESCRIPTION OF THE FIGURES

Useful embodiments of the invention shall now be described with reference to the attached figures. Similar elements or features are designated with the same reference signs in the figures. In the figures:
Fig. 1 shows a schematic cross-section through a human heart (4-chamber view);
Fig 2 shows a schematic representation of a sequence of medical images;
Fig. 3 is a diagram schematically illustrating the principle of a virtual multi-user collaboration according to an embodiment of the present invention
Fig. 4 shows a user interface according to an embodiment of the present invention
Fig. 5 shows a flow diagram illustrating the method according to an embodiment of the present invention.

### DESCRIPTION OF EMBODIMENTS

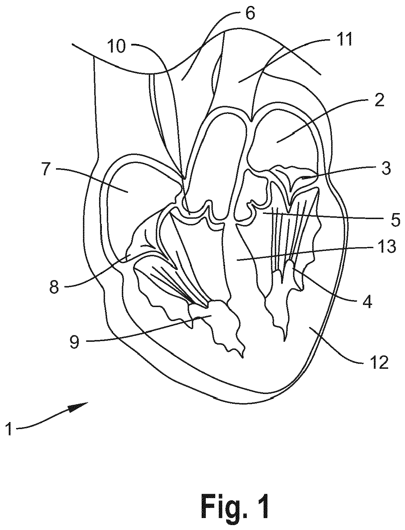

In order to better visualise the preferred application of the inventive method for analyzing medical image data in a virtual multi-user collaboration, FIG. 1 illustrates the structure of the human heart 1. The blood coming from the lungs flows into the left atrium 2 and from there through the mitral valve 3 into the left ventricle 4. From there, it is pumped through the aortic valve 5 into the aorta 6. This part is also termed left ventricular outflow tract (LVOT). The blood coming from the body flows into the right atrium 7 and is pumped through the tricuspid valve 8 into the right ventricle 9. From there, it is pumped through the pulmonary valve 10 into the pulmonary artery 11. Heart wall 12 is made of muscular tissue surrounding the heart chambers 2, 4, 7 and 9. The left and right ventricles are separated by the septum 13. It is evident from FIG. 1 that the heart has a complex shape, and in addition is constantly moving with the heartbeat, i.e. it is a dynamic anatomical structure. Thus, analyzing such complex structures such as the mitral valve 3 in order to plan a valve replacement is difficult and prone to errors. That is, the heart 1 in Fig. 1 represents an example of 3D medical image data achieved by MR, computed tomography (CT), position emission tomography (PET), ultrasound (US), or transoesophageal echocardiography (TEE). In order to achieve 4D medical image data, the time is used as the fourth dimension.

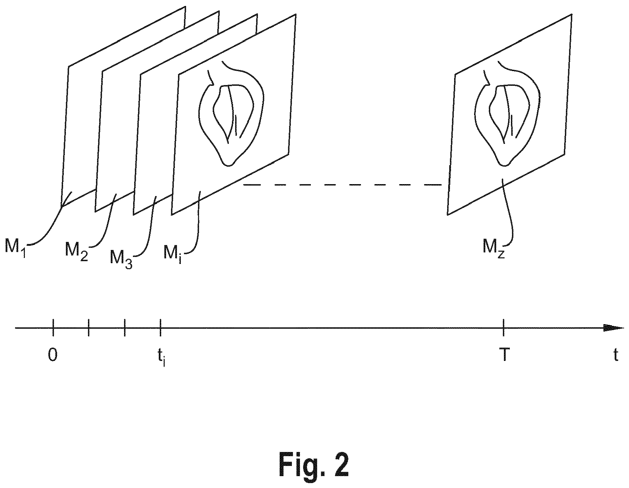

FIG. 2 shows a schematic representation of a sequence of ultrasound images M₁, M₂, M₃, ... Mz of the heart 1 based on medical image data. Z is the number of images acquired during one heart cycle, i.e. in time T, wherein T is about 0.5 to 1.5 seconds. For simplification, the figure shows two-dimensional images, however, according to the present invention a three-dimensional image and is acquired at each point in time tᵢ. A three-dimensional medical image may be formed by a stack of two-dimensional images. A four-dimensional medical image may be formed by a plurality of three-dimensional medical images that are consecutive displayed in a sequence. Such sequence of images M₁, M₂, M₃, ... Mz may be acquired for example by echocardiography of the moving heart, for example with a TEE probe. The sequence of medical images M₁, M₂, M₃, ... M_{Z} represent the 4D medical image data.

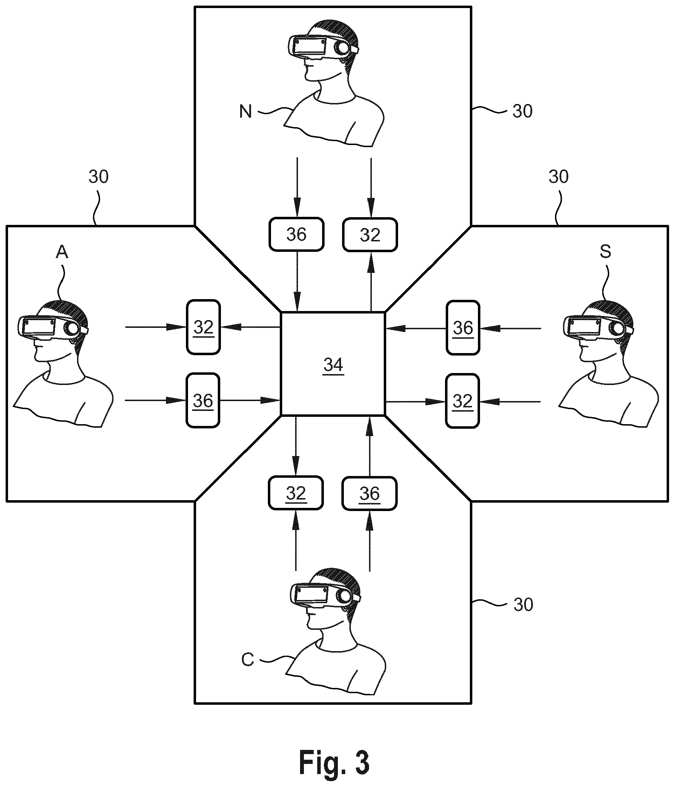

In one embodiment of the present invention, an interdisciplinary heart team (IHT) plans a heart surgery and is supposed to discuss a complex aspect previous to the actual surgery. In order to make sure all participants of the IHT have the same understanding of the patient anatomy and the planned procedure the team scheduled a virtual multi-user collaboration. During the collaboration each member of the team is at a different location. The IHT consists of: an interventional cardiologist C, a cardiac anesthesiologist A, an OR/cath lab nurse N and a cardiac surgeon S. Fig. 3 schematically shows the principle of the virtual multi-user collaboration according to the embodiment of the present invention.

As shown in Fig. 3, each participant/user has his/her own workspace WS. The 3D or 4D medical image data 34 is provided and loaded so as to be visualized in each workspace 30. As a result, an individual visualization 32 of the medical image data 34 is generated and provided for each workspace. In other words, each user has his/her own individual view 32 of the medical image data 34. Subsequently each of the users starts to work with the visualization. First each user changes the visualization so as to satisfy his/her requirements for analyzing the visualisation of the medical image data 34.

There are significant individual differences in the way different users view and analyse data sets (zoom levels, threshold/contrast/brightness settings, preferred viewing angles, etc.). Therefore, each user has his/her own isolated copy 32 of the visualization of the medical image data 34 and can resize, rotate, translate and set image settings using a controller 36. Nothing of this handling/view settings part is transferred to any other user. For example, the interventional cardiologist C prefers to show the visualization approximately 40 cm sized and 50 cm away from his head, a high threshold set so that leaflets of the mitral valve 4 disappear and a viewing direction from left atrium 2 through mitral valve 4 into left ventricle 4. On the other hand, the cardiac anesthesiologist A prefers to show the visualization as large as possible (100-120 cm) such that he/she can move his/her head into the visualization, a lower threshold set so that the leaflets are easily visible and a viewing direction from the side and in two-chamber view. Further, the OR/cath lab nurse N and the cardiac surgeon S prefers further individual views onto the visualisation. This changing of the visualisation 32 is carried out individually and independently by each user so as to obtain an individual visualization (i.e. an individual view of the medical image data). It is to be noted, that none of the users sees the changes of the individual view of the medical image data executed by the other users.

In addition, in case the medical image data changes due to new measurements that are available, the visualization which is based on the medical image data, is automatically updated. That is, in each workspace 30 the visualization is updated based on the medical image data that are shared by all users. Another reason why new medical image data are available is because a patient's organ is simultaneously analysed while at the same time the collaboration takes place and the acquired data are directly provided, in case the patient is an emergency, for example. Further, each user sees in his/her workspace 30 on which version of the medical image data the visualization is based. In addition, each user may individually change between different versions of visualizations.

Then the analyzing process starts and each user executes the analyzing process in his/her workspace 30 while having the individual view of the medical image data. That is, each user makes his/her own measurements/annotations which are results of the analyzing process within his/her own workspace 30 using the controller. In other words, each user controls the medical image data in that he/she adds the result of the analyzing process via his/her own workspace 30 directly into the medical image data. At the same time, the measurements/annotations inputted via the controller are transmitted to the medical image data shared by all users. Consequently, each user sees the measurements/annotations of the other users live within his/her own visualization of the medical image data. As a result, each user may immediately see and receive measurements/annotations of the other users a potentially use them for his/her own approach.

For example, the same image dataset appears for all 4 users simultaneously. Each user (C, A, N and S) makes his own measurements and annotations. Each user sees the annotations of the other users live at his own visualisation of the image dataset. As an example, the cardiologist C and the surgeon S may have different ideas about which procedure is right, but the anaesthesiologist A, after seeing both approaches, has another idea that combines the approaches of S and N in a meaningful way. When using the method of the present invention, S and N will make different measurements and annotations, both of which can be seen by A. A may thus annotate his improved proposal on his own visualisation of the image dataset. Every user sees his new annotation live on his own model/visualisation and agrees. Thereby, a more efficient meeting of an interdisciplinary heart is possible.

Since the measurements/annotations are directly transferred (i.e. synchronised with) to the medical image data 34 shared by all users, the measurements/annotations may hide or cover a part of the visualization in a workspace 30 of another user. Therefore, if in one user's workspace 30 a part of the visualization is hided and/or covers, the user may disable the respective measurement/annotation. Alternatively, the user may adjust the measurement/annotation so as to be transparent.

That is, according to the present invention, during a collaboration fewer unnecessary interactions, the sharing of measurements/annotations is faster and each user has an unrestricted view on the region of interest regardless of how many participants are in the VR session. Further, since individual understanding of anatomy differs, differently perceived reference points and structures in the medical 3D datasets inevitably lead to interobserver variability, different measurement (procedures), different assumptions and different interpretations of anatomical correlations. However, according to the present invention these phenomena is avoided because each user may individually change the visualization in that way, he/she prefers to work. In addition, the user now sees his and the changes of the other users live in his individual view, which he can handle and adjust according to his personal viewing preferences.

In another embodiment of the present invention, at least one workspace 30 is an AR workspace. In other words, the procedure is the same as that of the previous embodiment with the exception that the user sees both the visualization and the real environment, wherein the real environment forms a background for the visualization. In order to enable the user to sufficiently see and analyse the visualization, the AR-workspace, in this embodiment, has a virtual environment that is interposed between the visualization and the real environment. As a result, the real environment may be shadowed by increasing the opacity of the virtual environment. As a result, the AR-workspace 30 may automatically set the opacity/transparency of the virtual background so as to provide the target contrast within the workspace 30. The AR workplace is particularly useful when the user interacts with a person (e.g. a patient) in the real environment at the same time. Further, the virtual environment is automatically adapted to varying light conditions. Alternatively, in another embodiment each user may individually adjust the virtual environment in order to satisfy his/her own requirements and/or preferences.

According to a further embodiment, users can synchronize their individual view with another user, for example a teacher showing a critical measurement or positioning of a medical device to a classroom of students. That is, the individual view 32 towards the visualization is shared with another one or multiple participant of the collaboration. A user can put himself in the optimal viewing position and watch other (e.g., more experienced) users live and in real 3D while they make certain measurements, annotations or position device like artificial heart valves within the visualization to determine their optimal position. For example the "Teacher" can first observe (from the student's view) how the student would do a certain annotation/measurement and then synchronize the individual view of one or multiple students with his own individual view, e.g. if he wants to demonstrate a specific part of the intervention or measurement from his exact point of view. For this scenario one user would take over the moderation part, similar to a GoToMeeting ("Presenter/Teacher"), whereas all other participants ("Participants/Students") have a more limited functionality.

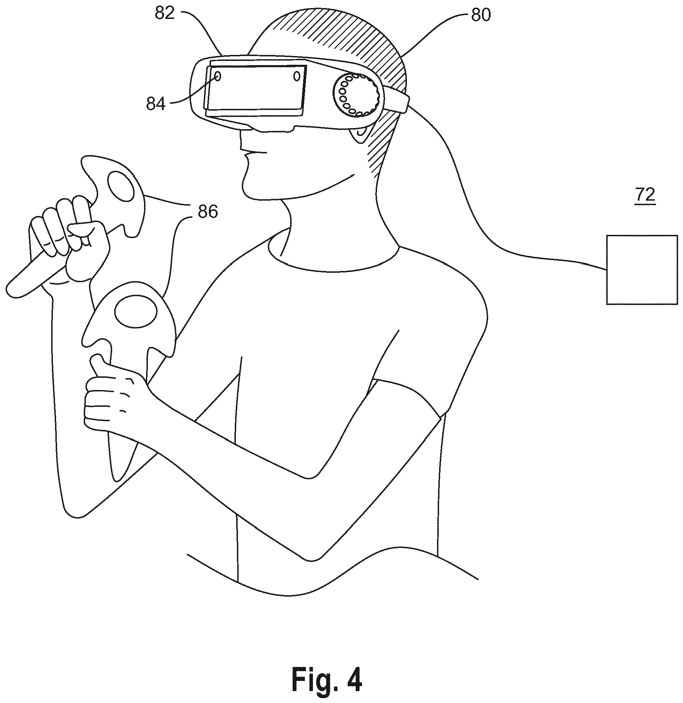

In a preferred embodiment, the user interface is a VR interface, as shown in FIG. 4. Such interface is realised by a virtual reality headset 82 worn by a user 80. The headset 82 is connected to a computer 72, either through a cable or through wireless connection. Such virtual reality headset 82 includes internal displays, separate for each eye, as well as position sensors 84 which track the movement of the head. Such headset may also include cameras, in case an augmented reality environment is to be presented. Further, the user 80 is holding VR controllers 86 in his hands, wherein the controllers 86 also include position sensors (not shown) as well as buttons or other input elements. Such virtual reality controller 86 allows a user to grab and move an object displayed in the virtual reality environment 50. The VR headset may for example be an HTC VIVE headset and corresponding VR controllers.

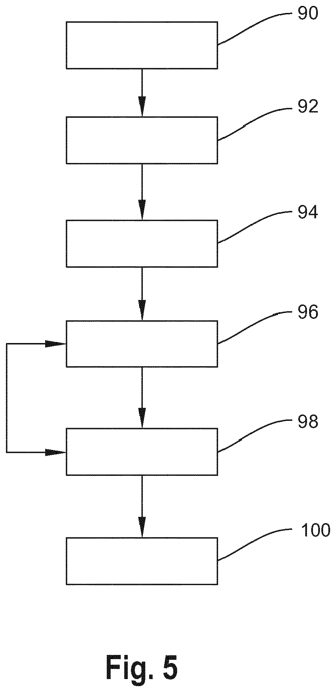

FIG. 5 shows a flow diagram illustrating the method according to an embodiment of the invention. In step 90, medical image data including 3D or 4D image information showing e.g. the moving heart are provided. In case the 4D medical image data are provided the sequence spanning a time period corresponding to one heartbeat. In step 92, the medical image data is loaded into the workspace 30 of each user so as to simultaneously display a visualization of the medical image data to each user, for example by generating a volume rendered or a surface rendered visualization. In step 94, each user is allowed to individually and independently from each other change the visualization of the medical image data, so as to obtain an individual visualization (i.e. his/her individual view of the medical image data) of the medical image data in each workspace 30 pertaining to each user. In step 96, at least one user is allowed to execute an analyzing process of the medical image data in his/her workspace 30. Such analyzing process includes making measurements and annotations which are positioned within the 3D coordinate system. In step 98 the result of the analyzing process is displayed in the workspace 30 in which the analyzing process was carried out. In step 100 the result of the analyzing process is synchronized in real-time with the at least one other workspace 30 such that each workspace 30 displays the result of the analyzing process in the respective individual visualization of the medical image data. The steps 96 to 100 are executed simultaneously, that is, the measurements and annotations made in step 96 are directly visibly in each of the workspaces because the measurements and annotations are directly implemented in the medical image data 34 shared by each user.

While the invention has been illustrated and described in detail in the drawings and foregoing description, such illustration and description are to be considered illustrative or exemplary and not descriptive; the invention is not limited to the disclosed embodiments.

## Claims

1. Method for analyzing medical image data (34) in a virtual multi-user collaboration, wherein
the medical image data (34) is analysed by at least two users (A, N,C, S),
each user having his/her own workspace (30), wherein the workspace (30) is a XR-Workspace,
the method including the steps of:
providing medical image data (34) including 3D or 4D image information,
loading the medical image data (34) into the workspace (30) of each user (A, N,C, S) so as to simultaneously display a visualization (32) of the medical image data (34) to each user,
allowing each user (A, N,C, S) to individually and independently from each other change the visualization (32) of the medical image data (34), so as to obtain an individual visualization (32) of the medical image data (34) in each workspace pertaining to each user,
allowing at least one user (A, N,C, S) to execute an analyzing process of the medical image data (34) in his/her workspace (30),
displaying the result of the analyzing process in the workspace (30) in which the analyzing process was carried out, and
synchronizing the result of the analyzing process in real-time with the at least one other workspace (30) such that each workspace (30) displays the result of the analyzing process in the respective individual visualization (32) of the medical image data (34).

2. Method for analyzing medical image data (34) in a virtual multi-user collaboration according to claim 1, wherein the displaying of the result of the analyzing process may be selectively and individually enabled and disabled by a user (A, N,C, S) in his/her workspace.

3. Method for analyzing medical image data (34) in a virtual multi-user collaboration according to claim 1 or 2,
wherein at least one workspace (30) is an AR- workspace or MR-workspace, and
wherein at least one visualisation parameter within the AR- or MR-workspace, in particular a transparency and/or a colour of the visualization (32) of the medical image data (34) and/or the result of the analyzing process is/are adjusted automatically, so as to allow the user (A, N,C, S) to view the visualization (32) of the medical image data (34) and/or the result of the analyzing process superposed on the real environment with a target contrast.

4. Method for analyzing medical image data (34) in a virtual multi-user collaboration according to any one of the preceding claims,
wherein each workspace (30) has its own virtual environment in which the visualization (32) of the medical image data (34) and the result of the analyzing process are displayed, and
wherein the method further includes the step of:
allowing each user (A, N,C, S) to individually and independently adjust a visualization (32) parameter of the virtual environment so as to adjust a contrast within the workspace (30), preferably by setting a transparency and/or a colour of the virtual environment.

5. Method for analyzing medical image data (34) in a virtual multi-user collaboration according to claim 4, wherein the virtual environment includes at least one virtual control element.

6. Method for analyzing medical image data (34) in a virtual multi-user collaboration according to any one of claims 1 to 5, wherein the step of allowing each user (A, N,C, S) individually and independently change the visualization (32) of the medical image data (34) includes the use of a controller (36) in order to execute the change of the visualization (32) using hand gestures, preferably by grabbing an object in the workspace (30).

7. Method for analyzing medical image data (34) in a virtual multi-user collaboration according to any one of claims 1 to 6, wherein the step of allowing each user (A, N,C, S) to individually and independently change the visualization (32) of the medical image data (34) includes manipulating the visualization (32) so as to rotate the visualization (32), cut away a part of the visualization (32), change rendering parameters of the visualization (32), change image settings of the visualization, change a contrast of the visualization, change voxel intensity thresholds of the visualization (32) and/or change a size of the visualization (32).

8. Method for analyzing medical image data (34) in a virtual multi-user collaboration according to any one of claims 1 to 7,
wherein at least one user (A, N,C, S) may adopt the change(s) of the visualization (32) of the medical image data (34) made by another user, or
wherein one user may force at least one other user to adopt his/her change(s) of the visualization (32) of the medical image data (34).

9. Method for analyzing medical image data (34) in a virtual multi-user collaboration according to any one of claims 1 to 8, wherein the step of allowing at least one user (A, N,C, S) to execute an analyzing process of the medical image data (34) in his/her workspace (30) further includes taking 3D measurements, executing MPR-Mode and/or inserting annotation.

10. Method for analyzing medical image data (34) in a virtual multi-user collaboration according to any one of claims 1 to 9, wherein the step of allowing at least one user (A, N,C, S) to execute the analyzing process of the medical image data (34) in his/her workspace (30) further includes positioning at least one model of a medical device, specifically an implant, within the visualization (32) of the medical image data (34) so as to determine its operational position.

11. Method for analyzing medical image data (34) in a virtual multi-user collaboration according to claim 10,
wherein the operational position of the model of the medical device is determined by visualizing the medical device dynamically in operation, preferably in combination with the 4D image information.

12. Computer program comprising program code instructions, which, when executed by a processor, enables the processor to carry out the method according to any one of claims 1 to 11.

13. User interface configured to be used in executing a method according to any one of claims 1 to 11, wherein the user interface includes:
a XR display device, in particular a VR headset (80), for displaying a visualization (32) of medical image data (34) and a result of an analyzing process in real-time within a workspace (30) to a user (A, N,C, S),
wherein the workspace (30) is a XR-workspace, and
wherein the workspace (30) includes a virtual environment so as to display the visualization (32) of medical image data (34) and the result of the analyzing within the virtual environment, and
a tracked controller (36) configured to be used during execution of the method in order to input commands by gestures of the user, wherein the commands include:
selectively and individually enable and disable the display of the result of the analyzing process.

14. User interface according to claim 13, wherein the commands further include:
individually and independently adjust a contrast within the workspace (30) of the user, preferably by setting a transparency and/or a colour of the virtual environment.

15. System for analyzing medical image data (34) in a virtual multi-user collaboration including:
a processor (72) configured to carry out the method according to any one of claims 1 to 11, and
at least two user interfaces according to claim 13 or 14 that are connected to the processor.
